# EUROPEAN PATENT APPLICATION

(11) **EP 2 305 830 A2**
(43) Date of publication of application: **06.04.2011**
(21) Application number: 10186765.3
(22) Date of filing: 06.06.2006
(51) Int. Cl.: C12Q 1/02, A61K 31/282, A61P 35/00, C12N 15/09, C12Q 1/68, G01N 33/15, G01N 33/50

(54) **Method for evaluation of compound using RSK1**

(30) Priority: 07.06.2005 JP 2005166412
(62) Divisional of application: 06747287.8
(71) Applicant: MSD K.K., Chiyoda-ku Tokyo 102-8667 (JP)
(72) Inventor: Mizuarai, Shinji, Tsukuba-shi Ibaraki 3002611 (JP); Kotani, Hidehito, Tsukuba-shi Ibaraki 3002611 (JP); Shimomura, Toshiyasu, Tsukuba-shi Ibaraki 3002611 (JP); Taniguchi, Eri, San Antonio, TX 78229 (US)
(74) Representative: Hussain, Deeba

(57) **Abstract**

A method for evaluation of a compound comprising the steps of introducing an RSKl gene into a cell to prepare a cell capable of expressing RSKl, contacting a compound to be evaluated with the cell, and detecting the specific binding of the compound to RSK1; and a compound given by the method. The compound can be used as a potentiator or cisplatin. The method enables to find a gene which acts specifically on a cell of cancer induced by the abnormality of p53 or enhanced MAPK pathway and to evaluate a compound by using the gene.

## Description

### TECHNICAL FIELD

The present invention relates to a method for evaluation of a compound using RSK1, and to a compound obtained according to the method.

### BACKGROUND ART

It is widely known that genetic abnormality is seen in cancer cells; and heretofore, many cancer genes and cancer suppressor genes have been found. In addition, it has been clarified that there exist multi-stage oncogenic mechanisms that require abnormality in multiple genes for oncogenic transformation of normal cells. Concretely, it is said that oncogenic transformation of normal cells requires accumulation of abnormality in multiple genes including DNA repair genes, cancer suppressor genes and cancer genes.

Above all, p53 known as a cancer suppressor gene was first found as a molecule to form a complex with a T-antigen of SV40 that is a tumor virus (Non-Patent Reference 1). After then, it has been clarified that the p53 gene is a cancer suppressor gene existing in the short arm of No. 17 chromosome (17p13) that is deleted in many cancers and both the gene and its allele are inactivated through deletion and mutation (Non-Patent Reference 2). Further, it has been clarified that the gene product is a transcriptional regulator comprising 393 amino acids, and this functions as forming a tetramer. Moreover, an extremely large number of genes have been identified as target genes for p53, including p21 and 14-3-3 that participate in cell cycle, and bax, PIG3 and GADD45 that participate in apoptosis; and it is considered that p53 abnormality may bring about the abnormality in transcriptional regulation of its target genes.

In fact, it has been clarified that the p53 gene mutation reaches 70% in lung cancer, 45% in stomach cancer, 30% in breast cancer, 65% in colon cancer, 61% in bladder cancer, and 70% in pancreas cancer (Non-Patent Reference 3); and the data are noticeably high as compared with those of other cancer suppressor genes.

In that situation, many studies have been made relating to the relationship between the mutation and dysfunction of p53 gene and diseases. For example, Soussi et al. made database of p53 mutation of 15000 cases or more (http://p53.curie.fr/: Non-Patent Reference 4), indicating frequent mutation occurring in the codons 175, 245, 248 and 273 positioned within the DNA-binding domain region of p53. In addition, it has been clarified that mutation from Arg to Ser is seen in the codon 249 in the DNA-binding domain in some types of hepatocellular carcinoma (Non-Patent Reference 5). Further, it has been reported that in the skin cancer caused by UV rays, CC in the p53 gene is thymine-dimerized (Non-Patent Reference 6). To that effect, p53 has great influences on the onset of many cancers, and therefore it is desired to identify potential drug target genes effective only in p53 abnormal cells.

On the other hand, RSK1 (SEQ ID NO 1: Accession No. NM 002953) is a molecule that is positioned downstream of a RAS/MAP kinase pathway and activated by ERK1/2 (Non-Patent Reference 7). In addition, it is known that RSK1 is activated in many primary tumors and established cell lines and is also activated by chemicals that induce DNA disorder.
(Non-Patent Reference 1) J. Virol., Vol. 31, p. 463, 1979
(Non-Patent Reference 2) N. Engl. J. Med., Vol. 319, p. 525, 1988
(Non-Patent Reference 3) Experimental Medicine, Vol. 19, p. 135, 2001
(Non-Patent Reference 4) Nucleic. Acids. Res., Vol. 22, p. 3551, 1994
(Non-Patent Reference 5) Nature, Vol. 350, p. 427, 1991
(Non-Patent Reference 6) PNAS, Vol. 88, p. 10124, 1991
(Non-Patent Reference 7) J. Biol. Chem.,, Vol. 273, p. 1496, 1998

### DISCLOSURE OF THE INVENTION

However, at present, there is known no information indicating the possibility that RSK1 could be an effective anticancer agent target gene specific to p53 mutation cells. In addition, at present, there is known little information relating to anticancer agent target genes specifically effective to cancer cells caused by p53 abnormality, and much more information is desired for potential drug target genes.

The invention has been made in consideration of the above-mentioned prior art problems, and its object is to find out an anticancer agent target gene specifically effective to cancer cells caused by p53 abnormality, and to enable compound evaluation by the use of the gene.

The present inventors have assiduously studied for the purpose of attaining the above object and, as a result, have found that, when RSK1 gene expression is inhibited in the cells having experienced abnormality in p53 expression, then the cell viability lowers, and have completed the invention.

Specifically, the method of compound evaluation of the invention is a method for evaluation of a compound effective for treatment of cancer, and is characterized by comprising a step of introducing an RSK1 gene into a cell to prepare a cell capable of expressing RSK1, a step of contacting a test compound with the cell, and a step of detecting the specific binding of the compound to RSK1. Use of the evaluation method enables evaluation and screening of compounds that may have some influence on the function of RSK1 through contact or binding to RSK1, such as RSK1 inhibitor.

Also, the compound evaluation method of the invention is a method for evaluation of a compound effective for treatment of cancer, and is characterized by comprising a step of introducing an RSK1 gene into a cell to prepare a cell capable of expressing RSK1, a step of contacting a test compound with the cell, a step of measuring the activity of the intracellular transmitter formed by the contact, and a step of comparing this activity with the activity of the intracellular transmitter in a case not contacted with the compound. Use of the evaluation method enables evaluation and screening of compounds that may have some influence on the intracellular transmittance via RSK1 through contact or binding to RSK1.

Also, the compound evaluation method of the invention is a method for evaluation of a compound effective for treatment of cancer, and is characterized by comprising a step of introducing an RSK1 gene into a cell to prepare a cell capable of expressing RSK1, a step of contacting a test compound with the cell, a step of measuring the expression level of RSK1 or the intracellular transmitter via RSK1, and a step of selecting the compound that has increased or decreased the expression level of RSK1 or the intracellular transmitter via RSK1, as compared with that in the case before the contact. Use of the evaluation method enables evaluation and screening of compounds that may have some influence on the expression of RSK1 or intracellular transmittance via RSK1 through contact or binding to RSK1.

Further, the compound evaluation method of the invention is a method for evaluation of a compound effective for treatment of cancer, and is characterized by comprising a step of contacting a test compound with RSK1, and a step of detecting the change in the activity of RSK1 through the contact. Use of the evaluation method enables construction of a compound evaluation system using a purified or recombined RSK1, and therefore enables evaluation or screening of compounds having some influence on the activity of RSK1 through contact or binding to RSK1 protein.

Also, the compound evaluation method of the invention is characterized in that the cancer is caused by p53 dysfunction. Use of the compound evaluation method enables evaluation or screening of compounds effective to cancer caused by p53 dysfunction.

Further, the compound evaluation method of the invention is characterized in that the cancer is a type of cancer with enhanced MAP kinase pathway. Use of the compound evaluation method enables evaluation or screening of compounds effective to cancer caused by enhanced MAP kinase pathway.

Also, the invention includes an RSK1 ligand isolated according to the compound evaluation method of the invention.

Further, the cisplatin enhancer of the invention is characterized by containing the RSK1 ligand. The enhancer makes it possible to enhance the potency of cisplatin, and makes it possible to reduce the necessary dose of cisplatin, therefore making it possible to relieve the side effect in cisplatin administration.

The method for administration of an anticancer agent of the invention is characterized by combined use of the above-mentioned RSK1 ligand and cisplatin. The administration method makes it possible to enhance the potency of cisplatin, and makes it possible to reduce the necessary dose of cisplatin, therefore making it possible to relieve the side effect in cisplatin administration.

Further, the molecular diagnostic method for cancer of the invention comprises a step of measuring the expression level of the RSK1 gene in a test tissue or a test cell, a step of comparing the expression level of the gene and the expression level of the corresponding gene in a normal tissue or a normal cell, and, as a result of the comparison, a step of judging as to whether or not the expression level of the gene in the test tissue or the test cell is significantly larger than the expression level of the gene in the normal tissue or the normal cell. The molecular diagnostic method for cancer enables specific diagnosis of cancer with enhanced RAS/MAPK pathway.

The present inventors have found out a gene RSK1 that specifically acts in p53 abnormality-caused cancer. At the same time, it has been clarified that there exists a correlation between the activity of RAS/MAPK pathway that is enhanced in many cancers, and the activation of RSK1. In other words, RSK1 has been found out as a gene specifically acting in a cell with malignant transformation owing to the enhancement of the RAS/MAPK pathway therein. Accordingly, by evaluating a compound having the gene, it is possible to evaluate and screen anticancer agents. In addition, combined use the compound obtained by the compound evaluation method and cisplatin enables administration of a drug having a more effective anticancer effect and treatment with it.

### BRIEF DESCRIPTION OF THE DRAWINGS

Fig. 1 is a graph of investigating the viability change of p53-positive TOV21G cells depending on the p53 expression level and the RSK1 expression level therein. In the drawing, D indicates a case of using control siRNA; and ■ indicates a case of using RSK1 siRNA.
Fig. 2 is a graph of investigating the viability change of p53-negative TOV21 G cells depending on the p53 expression level and the RSK1 expression level therein. In the drawing, D indicates a case of using control siRNA; and ■ indicates a case of using RSK1 siRNA.
Fig. 3 is a graph of measuring the viability change of p53-positive A549 cells depending on the p53 expression level and the RSK1 expression level therein. In the drawing, D indicates a case of using control siRNA; and ■ indicates a case of using RSK1 siRNA.
Fig. 4 is a graph of measuring the viability change of p53-negative A549 cells depending on the p53 expression level and the RSK1 expression level therein. In the drawing, D indicates a case of using control siRNA; and ■ indicates a case of using RSK1 siRNA.
Fig. 5 is a graph of measuring the viability change of p53-positive MCF7 cells depending on the p53 expression level and the RSK1 expression level therein. In the drawing, D indicates a case of using control siRNA; and ■ indicates a case of using RSK1 siRNA.
Fig. 6 is a graph of measuring the viability change of p53-negative MCF7 cells depending on the p53 expression level and the RSK1 expression level therein. In the drawing, D indicates a case of using control siRNA; and ■ indicates a case of using RSK1 siRNA.
Fig. 7 is a graph of measuring the viability change of p53-positive U2OS cells depending on the p53 expression level and the RSK1 expression level therein. In the drawing, D indicates a case of using control siRNA; and ■ indicates a case of using RSK1 siRNA.
Fig. 8 is a graph of measuring the viability change of p53-dominant negative U2OS cells depending on the p53 expression level and the RSK1 expression level therein. In the drawing, D indicates a case of using control siRNA; and ■ indicates a case of using RSK1 siRNA.
Fig. 9 shows a graph of measuring the RSK1 expression level and the cell viability change in two types of pancreas-derived cells (SU8686 cells with activated RAS/MAPK pathway, and inactivated PANC1 cells). The lower part is a drawing of detecting phosphorylated RSK1 in the cells.
Fig. 10 shows a graph of measuring the RSK1 expression level and the cell viability change in two types of bladder-derived cells (T24 cells with activated RAS/MAPK pathway, and inactivated J82 cells). The lower part is a drawing of detecting phosphorylated RSK1 in the cells.
Fig. 11 shows a graph of measuring the RSK1 expression level and the cell viability change in two types of stomach-derived cells (KATOIII cells with activated RAS/MAPK pathway, and inactivated NCIN87 cells). The lower part is a drawing of detecting phosphorylated RSK1 in the cells.
Fig. 12 is a graph showing the cell growth change depending on the presence or absence of forced expression of k-Ras and on the RSK1 expression level change.

### BEST MODE FOR CARRYING OUT THE INVENTION

Preferred embodiments of the invention are described in detail hereinunder.

The terms as referred to in the invention are described.

"RSK1" in the invention is not specifically limited in point of the origin of the biological species, including, for example, RSK1 derived from human, monkey, mouse, rat, canine or rabbit. Above all, preferred is a human RSK1 gene as the objects to which the compound to be evaluated is administered are humans.

The RSK1 gene in the invention includes any one with one or more base substitution, deletion, addition or insertion, so far as it has a biological function analogous to that of RSK1 and has a kinase activity. The gene may be any one that codes for the intended protein, not specifically limited in point of its sequence, but its homology is preferably at least 50%, more preferably at least 70%, even more preferably at least 80%, still more preferably at least 90% (for example, 91, 92, 93, 94, 95, 96, 97, 98 or 99% or more).

The RSK1 gene in the invention includes a nucleic acid that hybridizes with the RSK1 gene under a stringent condition. "Hybridize under a stringent condition" as referred to herein means that the two nucleic acid fragments hybridize with each other under the hybridization condition described in Molecular Cloning: A Laboratory Manual, 2nd Edition, Cold Spring Harbor (1989), 9.47-9.62 and 11.45-11.61. More concretely, for example, the process under the condition comprises 6.0 × SSC hybridization at about 45°C followed by 2.0 × SSC washing at 50°C. For stringency selection, the salt concentration in the washing step may fall within a range of from about 2.0 × SSC at 50°C of low stringency to about 0.2 × SSC at 50°C of high stringency. Further, the temperature in the washing step may be elevated from room temperature of about 22°C under a low stringency condition up to about 65°C under a high stringency condition.

The RSK1 protein in the invention includes any one with one or more base substitution, deletion, addition or insertion, so far as it has a biological function analogous to that of RSK1 and has a kinase activity. The protein sequence is not specifically limited, but its homology is preferably at least 50%, more preferably at least 70%, even more preferably at least 80%, still more preferably at least 90% (for example, 91, 92, 93, 94, 95, 96, 97, 98 or 99% or more).

RSK1 is known as a kinase positioned downstream of a RAS/MAPK pathway, but the present inventors have found that RSK1 directly participates in p53 expression or RAS/MAPK pathway enhancement. The p53 expression level has close relation to malignant transformation of cells, and the RAS/MAPK pathway enhancement also has close relation to abnormal cell growth, or that is, malignant transformation. Accordingly, by measuring the expression level and the activity of RSK1, it may be possible to detect cancer based on the abnormality in p53 or Ras/MAPK pathway. In addition, since RSK1 has direct relation to p53 dysfunction or RAS/MAPK pathway enhancement, RSK1 has close relation to malignant transformation of cells. Accordingly, taking RSK1 as a potential drug target, the search for compounds acting on RSK1 will make it possible to obtain anticancer agents.

"p53 dysfunction" as referred to in the invention may be caused by base deletion (for example, nonsense mutation), substitution (for example, missense mutation, point mutation), insertion, frame shifting or the like in p53 gene, but the cause of dysfunction to which the invention is directed is not specifically limited, and includes any and every dysfunction caused by any of these.

Examples of p53 dysfunction include a case of p53 protein transcriptional activity reduction or activation, and a case with no transcription, further including a case of gene encoding mutation though no abnormality is recognized in the gene. Concretely, for example, it includes Li-Fraumeni syndrome (Science, Vol. 250, p. 1233, 1990), hepatocellular carcinoma (Nature, Vol. 350, p. 377, 1991), osteogenic sarcoma (Proc. Natl. Acad. Sci. USA, Vol. 84, p. 7716, 1987), rhabdomyosarcoma (Proc. Natl. Acad. Sci. USA, Vol. 87, p. 5863, 1990), colon cancer (Science, Vol. 244, p. 217, 1989), lung cancer (Science, Vol. 246, p. 491, 1989), glioblastoma (Am. J. Hum. Genet., Vol. 47 (suppl.), A4, 1990), esophageal cancer (Proc. Natl. Acad. Sci. USA, Vol. 87, p. 9958, 1990), bladder cancer (Science, Vol. 252, p. 706, 1991), squamous cell cancer (Proc. Natl. Acad. Sci. USA, Vol. 88, p. 10124, 1991), cervical cancer (Lancet, Vol. 339, p. 1070, 1992), lung cancer (Proc. Natl. Acad. Sci. USA, Vol. 89, p. 7262, 1992) and leukemia/lymphoma (J. Clin. Invest., Vol. 90, p. 653, 1992), in which p53 participation is suggested.

"RAS/MAPK pathway enhancement" in the invention means that the signal transmission in RAS/MAPK pathway is activated. The pathway transmits cell growth/differentiation signals even in normal cells, but in cancer tissues, the cells are in a condition of abnormal growth. Accordingly, the RAS/MAPK pathway enhancement in the invention means that, as compared with that in normal cells, the pathway is more activated.

"Test tissue" as referred to in the invention means a tissue that may be taken out of a biological object for cancer examination, and it may be any cancer tissue that must be investigated for p53 participation therein or may also be any other tissue that requires cancer diagnosis, not specifically limited in point of its type. Examples of the tissue include tissues derived from, for example, neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, chorionic disorder, vaginal cancer, ovarian cancer, fallopian tube cancer, ovarian germ cell tumor, skin cancer, myocosis fungoides, malignant melanoma, soft tissue sarcoma, bone neoplasm, malignant lymphoma, leukemia, myelodysplastic syndrome, multiple myeloma, lymphedema.

Similarly, "test cell" as referred to in the invention means a cell that may be taken out of a biological object for cancer examination, and it may be any cancer tissue-derived cell that must be investigated for p53 participation therein or may also be any other tissue-derived cell that requires cancer diagnosis, not specifically limited in point of its type. Examples of the tissue include cells derived from, for example, neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, chorionic disorder, vaginal cancer, ovarian cancer, fallopian tube cancer, ovarian germ cell tumor, skin cancer, myocosis fungoides, malignant melanoma, soft tissue sarcoma, bone neoplasm, malignant lymphoma, leukemia, myelodysplastic syndrome, multiple myeloma, lymphedema.

### (1) Compound Evaluation:

Using an RSK1 gene or protein, it is possible to evaluate a compound acting on RSK1. For detecting the effect to RSK1, employable are a method of detecting specific binding to RSK1 of a test compound (for example, binding to induce inhibition of enzymatic activity); a method of detecting the expression level of the gene having been converted through its contact with a test compound; and a method of detecting the activity of intracellular transmission caused through the contact. These are described below in order.

First described is the method of detecting the specific binding to RSK1 of a test compound to thereby evaluate the test compound.

The compound evaluation method of the invention is characterized by comprising a step of introducing an RSK1 gene into a cell to prepare a cell capable of expressing RSK1, a step of contacting a test compound with the cell, and a step of detecting the specific binding of the compound to RSK1.

The second method for compound evaluation of the invention is characterized by comprising a step of introducing an RSK1 gene into a cell to prepare a cell capable of expressing RSK1, a step of contacting a test compound with the cell, a step of measuring the activity of the intracellular transmitter formed by the contact, and a step of comparing this activity with the activity of the intracellular transmitter in a case not contacted with the test compound.

Not specifically limited, the test compound includes, for example, single compounds such as natural compounds, organic compounds, inorganic compounds, proteins, peptides, as well as expression products of compound library or gene library, cell extracts, cell culture supernatants, fermented microorganism products, marine organism extracts, plant extracts, procaryotic cell extracts, eucaryotic cell extracts and animal cell extracts. The test sample may be suitably labeled, if desired. The labeling includes, for example, radiolabeling, fluorescence activation. In addition to the above-mentioned test samples, the test compound further includes mixture of two or more of such test samples.

The RSK1 gene-expressing cell may be prepared according to a method known to those skilled in the art, and its concrete method is not specifically limited. For example, the cell may be prepared as follows: An RSK1 gene or a nucleic acid comprising a part of it is cloned into an expression vector containing a suitable promoter and a suitable transcriptional regulator, and the vector having the cloned nucleic acid is introduced into a host cell, thereby preparing the intended cell. In this, the vector may be any one usable as an expression vector, not specifically limited, and, for example, it includes pCMV-Tag, pcDNA3.1, pBlueBacHis2, pCI-neo, pcDNAI, pMClneo, pXT1, pSG5, pEF1/V5-HisB, pCR2.1, pET11, λgt11 and pCR3.1.

Next, the expression vector having, as introduced thereinto, the RSK1 gene or the nucleic acid comprising a part of it is introduced into a host cell. Not specifically limited, the host cell may be any one generally used for gene expression, including, for example, animal cells, insect cells, plant cells, microorganisms. Concretely, for example, they include COS1, COS7, CHO, NIH/3T3, 293, Raji, CV11, C1271, MRC-5, CPAE, HeLa, 293T and Sf9. For introducing the expression vector into a host cell, employable is any known method with no specific limitation. Concretely, for example, employable are an electroporation method, a calcium phosphate method, a DEAE-dextran method, a lipofection method, and a gene gun method.

Next, the RSK1-expression cell, thus prepared, is contacted with a test compound. The contact method is not specifically limited, and for example, a test sample may be added to a pure cell sample in which RSK1 is in a pure condition. When RSK1 is expressed in the cell or is expressed in the cell extract, then a test sample may be added to the cell culture or the cell extract. When the test sample is a protein, then, for example, a vector that contains a DNA coding for the protein may be introduced into the RSK1-expressing cell; or the vector may be added to the RSK1-expressing cell extract.

The binding of RSK1 to the test compound may be detected, for example, through detection of the labeling given to the RSK1-binding compound (for example, through detection of the binding amount based on radioactivity or fluorescence intensity); through detection of the index that the test compound inhibits the phosphorylation of the substrate peptide by RSK1 (for example, through detection of the phosphorylated amount by the use of radioactivity or fluorescence intensity); or through detection of the index of inhibition of signal transmission induced by the binding of RSK1 to the test compound (for example, induction of phosphorylation or apoptosis of substrate protein by BAD, IkB, Myt1 or TS1/2). It may also be detected based on the index of the expression level or the activity of the molecule (including RSK1) on the signal transmission pathway induced by the above-mentioned signal transmission. In this, when the binding is detected based on the index of the expression level, then the method for measuring the expressing level is not specifically limited. For example, it may be measured through northern blotting or western blotting or by the use of a DNA chip. The "expression level" as referred to in the invention is meant to indicate the absolute amount or the relative amount of the transcriptional product of the gene that codes for the protein existing on the transmission pathway via RSK1. In this case, the gene includes any of DNA or mRNA. In case where the object for expression detection is a protein, the "expression level" is meant to indicates the absolute amount or the relative amount of the translation product of the protein existing on the transmission pathway via RSK1. In case where the activity of the molecule on the signal transmission is taken as the index, then the activity measurement method is not specifically limited. Depending on the type of the molecule to be measured, a suitable method for it may be selected.

On the other hand, an isolated RSK1 protein may be directly used for compound evaluation. In other words, a test compound is contacted with an RSK1 protein, and then the change in the activity of the RSK1 protein produced through the contact is detected.

The contact method is not specifically limited. Concretely, for example, they may be mixed in a solution such as buffer (e.g., phosphate buffer) and may be contacted with each other; or an RSK1 protein may be fixed on a membrane, and it may be contacted with a test compound on the membrane.

Next, the change in the activity of RSK1 induced by the contact is detected.

For measuring the protein activity, a desired method may be selected depending on the property of the protein sued. Concretely, for example, a substrate suitable for RSK1 is prepared, and kinase assay is carried out in the presence of RSK1, the substrate and a test compound in a solvent. In this case, the enzymatic reaction may be confirmed by the intake of radiolabeled phosphorus (e.g., ³²P).

As in the above, as a result of evaluation of a compound according to the compound evaluation method of the invention, in case where the RSK1 activity in the presence of the test compound is lower than the binding activity (control) in the absence of the test compound, then it may be judged that the test compound is an antagonist having an activity of inhibiting the binding of RSK1 to the ligand in the invention. The antagonist inhibits the biological activity of the ligand to RSK1 and its analogue. Accordingly, the antagonist is useful as a pharmaceutical composition for treatment of cancer caused by the abnormality in the signal transmission system via RSK1, based on p53 dysfunction or RAS/MAPK pathway enhancement.

According to the compound evaluation method of the invention, it is possible to screen a compound that promotes or inhibits the intracellular signal transmission after binding of the test compound to RSK1. In other words, by evaluating plural test compounds according to the above-mentioned method, a compound capable of functioning as an agonist or an antagonist may be selected. As a result of the selection, in case where the change is inhibited as compared with the change in the signal transmission in the downstream after acting with the ligand or its analogue in the absence of the test compound, then it is judged that the test compound is a compound that inhibits the signal transmission to the downstream after binding of the test compound to RSK1. On the contrary, when the test compound enhances the intracellular signal transmission, then it may be judged that the test compound is a compound that promotes the intracellular signal transmission after the binding of the test compound to RSK1. The compound selected according to the screening method is effective for treatment and diagnosis of cancer based on p53 dysfunction or RAS/MAPK pathway enhancement.

According to the above-mentioned compound evaluation method of the invention, it is possible to evaluate a ligand for use in PET (positron emission tomography). PET is a method of noninvasive observation of biological function by administering to a living object, a radio-labeled ligand to a substance existing in the living body such as water, oxygen, glucose or amino acid or for the intended receptor, and this is utilized in studies and clinical medicine. PET is characterized in that it enables function-specific imaging, depending on the ligand used as a tracer; and the development of the tracer is indispensable for clarification of unknown biological functions and for diagnosis of diseases. According to the compound evaluation method of the invention, a PET ligand candidate substance may be used as the test compound, thereby enabling in-vitro evaluation of the substance.

### (2) RSK1 Ligand and Cisplatin Enhancer:

According to the compound evaluation method of the invention described in (1), an RSK1 ligand may be isolated. The ligand may be used as a drug having an anticancer effect by itself, and in addition, it may be used as a cisplatin enhancer. As the RSK1 ligand of the type, for example, mentioned is SL0101-1 (US 2005/0233985).

In this, cisplatin is a platinum complex having the following properties, and is used as an anticancer agent. In Japan, it is applied to lung cancer, head and neck cancer and esophageal cancer, and as its side effects, known are nausea, vomiting, renal toxicity, ear toxicity, and bone marrow depression.
Chemical name: cisplatin
Abbreviation: CDDP
Chemical formula: H₆ Cl₂ N₂ Pt
Molecular weight: 300.05
CAS registration number: 15663-27-1

As demonstrated in Examples mentioned below, the present inventors have clarified that cisplatin administration with inhibiting RSK1 expression significantly lowers the viability of cancer cells. This indicates a fact that the combined use of an RSK1 inhibitor and cisplatin has a remarkable anticancer effect. In addition, combined with an RSK1 inhibitor, the dose of cisplatin may be reduced to thereby relieve the side effect of cisplatin. In other words, an RSK1 inhibitor may be used as an enhancer for cisplatin.

The above-mentioned "combined use" means administration of two or more different types of drugs simultaneously or at different times. The administration route and the administration dose are not specifically limited, for which suitable conditions may be settled depending on the drugs.

The test compound to be such an RSK1 ligand or cisplatin enhancer is not specifically limited, including, for example, single compounds such as natural compounds, organic compounds, inorganic compounds, proteins, peptides, as well as RSK1 antibody, antisense, RNAi and ribozyme.

In case where the RSK1 ligand or cisplatin enhancer of the invention is used as medicines for human and other animals, the substance itself may be directly administered to the patients, or it may be formulated into pharmaceutical compositions according to known pharmaceutical preparation methods and may be administered to them. For example, the compositions may be used orally, as optionally sugar-coated tablets, capsules, elixirs or microcapsules; or may be used parenterally as germ-free solution or suspension injections with water or with any other pharmaceutically-acceptable liquid. For example, the ligand or enhancer may be suitably combined with a pharmaceutically-acceptable carrier or medium, concretely, germ-free water, physiological saline water, vegetable oil, emulsifier, suspending agent, surfactant, stabilizer, flavor, excipient, vehicle, preservative, binder and the like, and may be mixed as a unit dosage form required in generally-admitted pharmaceutical preparation, thereby producing pharmaceutical compositions containing it.

The additive capable of being mixed in the tablets and capsules are, for example, binder such as gelatin, corn starch, tragacanth gum, gum arabic; excipient such as crystalline cellulose; expander such as corn starch, gelatin, alginic acid; lubricant such as magnesium stearate; sweetener such as sucrose, lactose, saccharin; flavor such as peppermint, akamono oil and cherry. The capsules as preparation unit forms can contain liquid carriers such as fats and oils in addition to the above materials. The germ-free compositons for injection may be formulated according to ordinary pharmaceutical preparation using a vehicle such as distilled water for injection.

The aqueous solution for injection includes, for example, isotonic liquid containing physiological saline water, glucose and any other auxiliary agent, for example, D-sorbitol, D-mannose, D-mannitol, sodium chloride; and it may be combined with a suitable dissolution promoter, for example, alcohol, concretely ethanol, polyalcohol such as propylene glycol, polyethylene glycol, as well as nonionic surfactant, such as Polysorbate 80^{(™)}, HCO-50.

The oily liquid includes sesame oil, soybean oil, and it may be combined with benzyl benzoate or benzyl alcohol serving as a dissolution promoter. In addition, the composition may further contain buffer, such as phosphate buffer, sodium acetate buffer; analgesic agent such as procaine chloride; stabilizer such as benzyl alcohol, phenol; and antioxidant. Thus prepared, the injection is generally filled in suitable ampoules.

Administration to patients may be effected in any method known to those skilled in the art, for example as intra-arterial injection, intravenous injection or subcutaneous injection, or intranasal, transbronchial, intramuscular, percutaneous or oral administration. The dose may vary, depending on the body weight and the age of patients, and the administration route, but may be any suitable one known to those skilled in the art. When the compound is capable of encoded by DNA, the DNA may be inserted into a vector for gene therapy and may be used for gene therapy. The dose and the administration method may vary depending on the body weight, the age and the condition of patients, but may be suitably selected by anyone skilled in the art.

Though varying depending on the condition, the compound dose may be as follows: In oral administration, the dose may be from about 0.1 to 100 mg, preferably from about 1.0 to 50 mg a day, more preferably from 1.0 to 20 mg to an adult (having a body weight of 60 kg).

In parenteral administration, the dose per one time may differ depending on the object for administration, the targeted organ, the condition and the administration route. For example, as an injection, the dose may be generally from about 0.01 to 30 mg a day, preferably from about 0.1 to 20 mg, more preferably from about 0.1 to 10 mg or so to an adult (having a body weight of 60 kg), and intravenous injection with the dose may be more advantageous.

Regarding the administration condition for cisplatin, its dose is known and it may be administered in a known administration method. When combined with an RSK1 inhibitor, the dose of cisplatin may be smaller than a standard dose thereof. Accordingly, the side effect caused by cisplatin may be inhibited.

### (3) Method of Molecular Diagnosis of Cancer:

In the invention, the expression level of RSK1 gene is first measured in the test tissue or the test cell. The method for measuring the expression level of the gene is not specifically limited. For example, employable are a method of RT-PCR using the genomic DNA extracted from a test tissue or a test cell, as a template; a method of using a microarray plotted with the gene; and a northern blotting method. The "expression level" of gene as referred to herein is meant to indicate the absolute amount or the relative amount of the gene transcriptional product. Regarding the relative amount, the expression level of the gene may be determined in relative comparison with the expression level thereof in the normal tissue mentioned hereinunder.

Next, the gene expression level measured according to the above method is compared with the expression level of the corresponding gene in a normal tissue or a normal cell.

"Normal tissue or normal cell" as referred to herein is not specifically limited in point of its origin, so far as it is a tissue or a cell of a subject to be compared with the test tissue or the test cell; and it may be a healthy person-derived one, or a cancer patient-derived one. It may be a normal tissue or a normal cell existing around a cancer tissue.

In this step, the expression level of the RSK1 gene expressed in the test tissue or cell is compared with the expression level of the RSK1 gene (corresponding gene) expressed in the normal tissue or cell, for which, the absolute amount of the expression level may be compared with each other, or the relative value may be computed through comparison.

Next, as a result of the comparison, it is determined whether or not the expression level of the gene in the test tissue or the test cell is significantly higher than that of the gene in the normal tissue or the normal cell.

The method for determination of the significant difference is not specifically limited, for which employable is a statistical test method known to those skilled in the art.

As a result of comparison between the expression level of the gene in the test tissue or the test cell and that in the normal tissue or the normal cell, in case where the expression in the test tissue or the test cell is recognized higher with a significant difference therebetween, then it may be determined that the RAS/MAPK pathway is enhanced in the test tissue or the test cell, and the tissue or the cell may be in malignant transformation. Accordingly, the method enables diagnosis of cancer that is known to have relation to RAS/MAPK pathway enhancement (for example, see Nature Reviews Cancer, Vol. 4, p. 937, 2004).

### (Examples)

The invention is described more concretely with reference to the following Examples, to which, however, the invention should not be limited.

### (Example 1)

The following experiment was carried out for investigating, in cells in which p53 is positive (p53 is functioning) or negative (p53 is not functioning), what influence RSK1 may have on the existence of viability those cells.

First, as p53-positive cells, prepared were TOV21G, A549, MCF7 and U2OS cells. As p53-negative cells, prepared were TOV21G-shp53, A549-shp53, MCF7-shp53 and U20S-shp53 by introducing shRNA of p53 into the above-mentioned four types of cells. Further, dominant negative U20S-mtp53 cells were also prepared by introducing V157Fp53 into U2OS cells.

Next, siRNA of RSK1 was introduced into the above cells according to a lipofection method, and the cells were incubated in a 10% FCS-containing D-MEM medium (10% FCS-containing MacCoy's 5A medium for U2OS cells) in the presence or absence of cisplatin (CDDP) for 96 hours, and then their viability was measured through MTT assay. As the siRNA of RSK1, used was a mixture of the following three sequences.
5'-CUGUCAAGGUCAUUGAUAATT-3' (SEQ ID NO 2)
5'-CCAAGGACCUGGUGUCCAATT-3' (SEQ ID NO 3)
5'-CAGUGACACACCAGAGGAATT-3' (SEQ ID NO 4)

It was confirmed that at least 90% of RSK1 expression was inhibited in the cells.

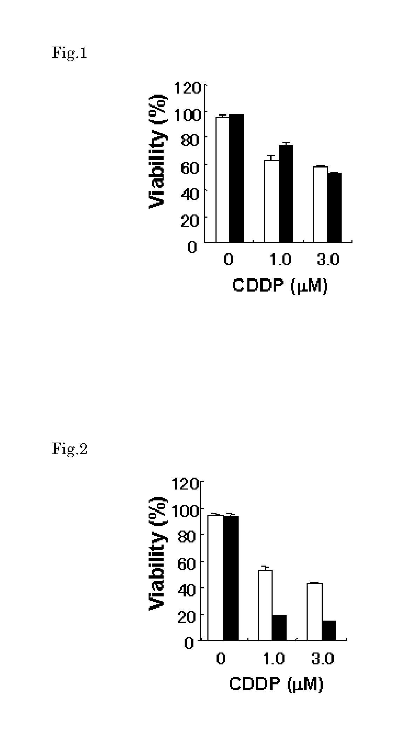

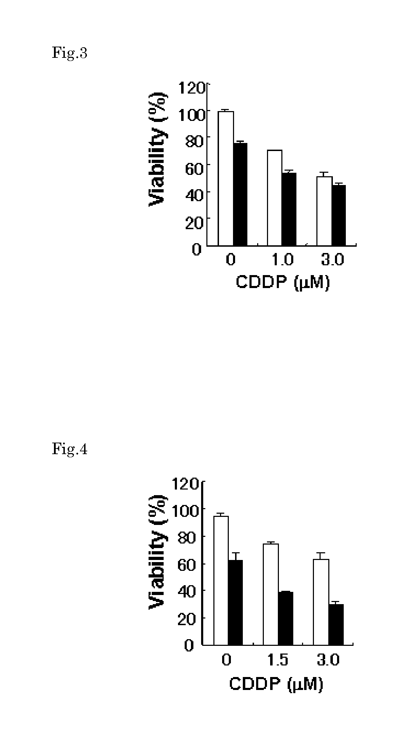

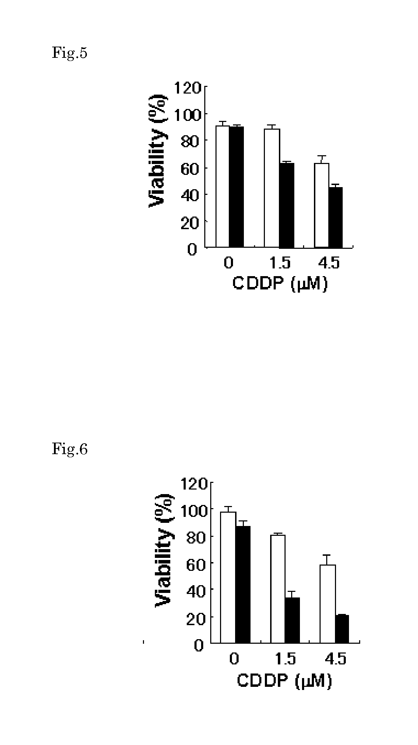

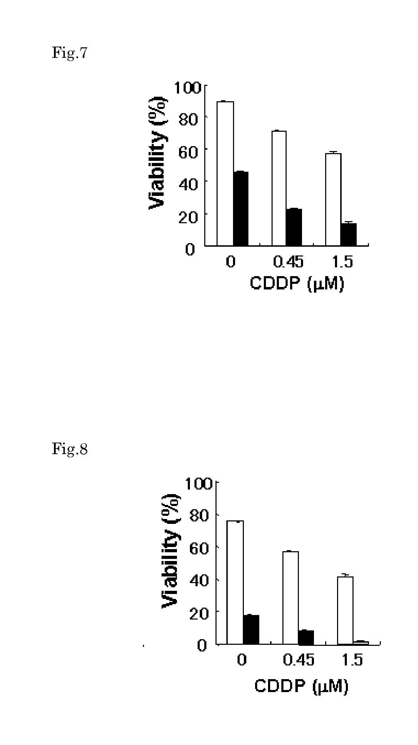

As in Fig. 1 to Fig. 8, the RSK1 expression inhibition did not have any significant influence on the cell viability. However, in the p53-negative cells, the effect of cisplatin was enhanced and the cell viability was greatly reduced. These results indicate that RSK1 has a stronger influence on p53-negative cells than on p53-positive cells. In other words, it may be determined that RSK1 could be a potential drug target for anticancer agents directed to cancer cells with p53 expression abnormality.

### (Example 2)

It is known that RAS/MAPK pathway activates cell growth promotion and apoptosis reduction in many cancer cells, and has close relation to activation of RSK1. Accordingly, the inventors made a hypothesis that the influence of RSK1 inhibition on RSK1-inactivated cells may be greater than on RSK1-activated cells, and investigated it.

First, by confirming the RSK1 phosphorylation level, RSK1-activated cells and inactivated cells were identified. Specifically, M-PER (by Pierce) was added to cells washed with PBS, and dissolved them. The sample corresponding to 20 µg of the whole protein was developed through SDS-PAGE, and transcribed onto a PVDF membrane. The RSK1 phosphorylation was confirmed through western blotting using a phosphorylation RSK1 (Thr359/Ser363) antibody, thereby obtaining SUS8686, T24 and KATOIII cells as RSK1-activated cells. Similarly, PANK1, J82 and NCI-N87 cells were obtained as RSK1-inactivated cells.

Next, siRNA of RSK1 was introduced into these cells, in which about 80% RSK1 expression inhibition was confirmed. Next, the cell viability was measured through MTT assay.

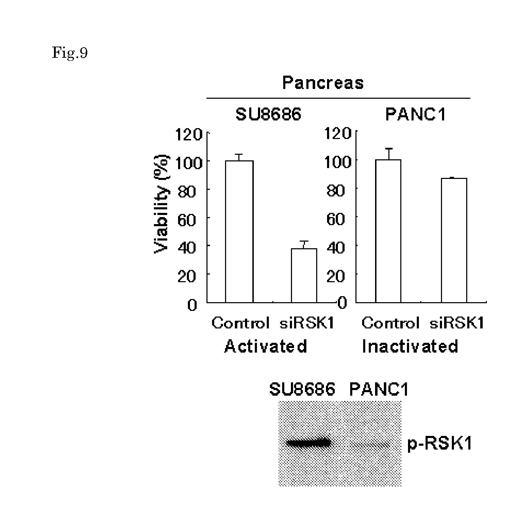

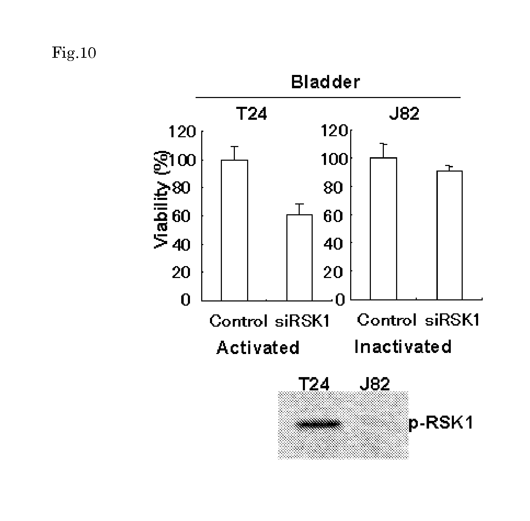

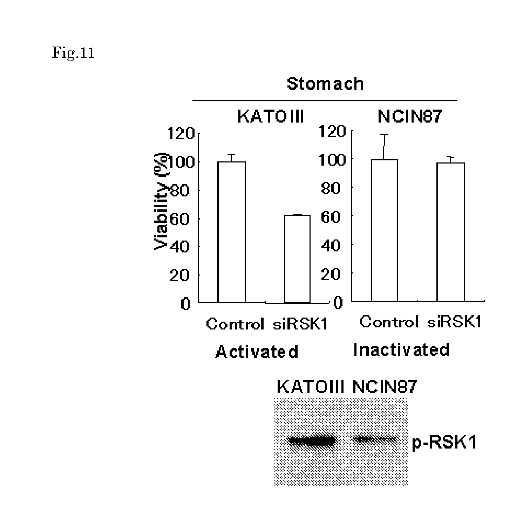

As in Figs. 9 to 11, it was confirmed that the RSK1 expression inhibition has a greater influence on the cell viability of RSK1-activated cells than on the cell viability of RSK1-inactivated cells. In other words, this suggests that the RSK1 expression may have a great influence on MAPK/RSK1-activated tumors.

### (Example 3)

The relationship between Ras/MAPK pathway and RSK1 was investigated.

First, using a medium RPMI-1604, k-Ras transformant cells, NIH3T3/k-Ras (1000 cells) were cultured on a 96-well plate. Simultaneously with the cultivation thereof, the cells were processed with 4 nM RSK1 siRNA (Dharmacon, RPS6KA1, siGENOME SMART pool) or Luc siRNA (as control), using siLentFect^{™} Lipid (by Bio-Rad).

The cell viability was measured in 72 hours after the above treatment, using CellTiter-Glo^{™} Luminescent Cell Viability Assay (by Promega). For measuring the RSK1 expression level, the sample was subjected to TaqMan RT-PCR in 24 hours after the above treatment. As the probe in TaqMan RT-PCR, used was Rsk probe (by Applied Biosystem); and as a control probe, used was 18S rRNA probe (by Applied Biosystem).

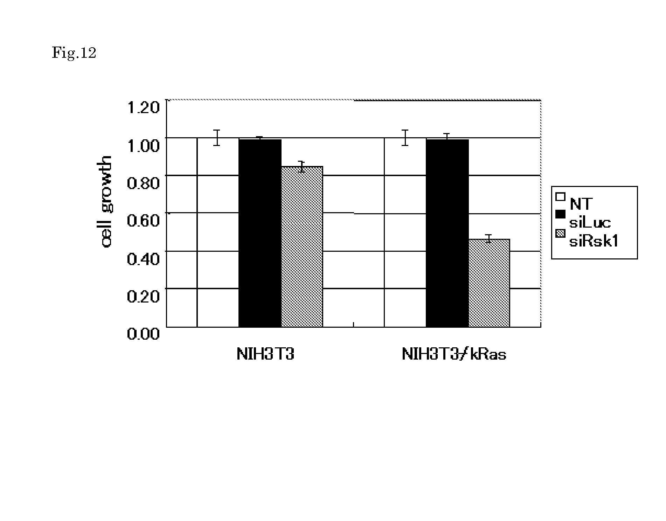

As in Fig. 12, the cell growth of the RSK1 expression-inhibited NIH3T3/kRas cells was inhibited by 53%; however, the cell growth inhibition of the RSK1 expression-inhibited NIH3T3 cells was only 14%. As compared with that of the control (processed with Luc siRNA), the cell growth of the RSK1 siRNA-processed NIH3T3/k-Ras was significantly reduced. Accordingly, it was confirmed that the RSK1 expression reduction in the Ras/MAPK pathway-enhanced cells results in the growth inhibition of the cells.

### INDUSTRIAL APPLICABILITY

RSK1 has been found as a target gene specifically acting on p53 abnormality-caused cancers, and the use of the gene enables compound evaluation. In addition, RSK1 has been found out as a target gene effectively acting on RAS/MAPK pathway-enhanced cancer cells, and the use of the gene enables compound evaluation. Further, combined use of the compound obtained according to the compound evaluation method and cisplatin enables administration of a drug having a more effective anticancer agent and treatment with it.

## Claims

1. A method for evaluation of a compound effective for treatment of cancer, **characterized by** comprising:
a step of preparing a cell expressing RSK1 by introducing an RSK1 gene,
a step of contacting a test compound with the cell, and
a step of detecting the specific binding of the compound to RSK1.

2. A method for evaluation of a compound effective for treatment of cancer, **characterized by** comprising:
a step of preparing a cell expressing RSK1 by introducing an RSK1 gene,
a step of contacting a test compound with the cell,
a step of measuring the activity of the intracellular transmitter formed by the contact, and
a step of comparing this activity with the activity of the intracellular transmitter in a case not contacted with the test compound.

3. A method for evaluation of a compound effective for treatment of cancer, **characterized by** comprising:
a step of preparing a cell expressing RSK1 by introducing an RSK1 gene,
a step of contacting a test compound with the cell,
a step of measuring the expression level of RSK1 or the intracellular transmitter via RSK1, and
a step of selecting the test compound that has increased or decreased the expression level of RSK1 or the intracellular transmitter via RSK1, as compared with that in the case before the contact.

4. A method for evaluation of a compound effective for treatment of cancer, **characterized by** comprising:
a step of contacting a test compound with RSK1, and
a step of detecting the change in the activity of RSK1 through the contact.

5. The compound evaluation method as claimed in claims 1 to 4, wherein the cancer is caused by p53 dysfunction.

6. The compound evaluation method as claimed in claims 1 to 5, wherein the cancer is a type of cancer with enhanced MAP kinase pathway.

7. An RSK1 ligand isolated according to the compound evaluation method of any one of claims 1 to 6.

8. A cisplatin enhancer containing the RSK1 ligand of claim 7.

9. A combination of a RSK1 ligand of claim 7 and cisplatin for simultaneous, separate or sequential administration.

10. A molecular diagnostic method for cancer, comprising:
a step of measuring the expression level of the RSK1 gene in a test tissue or a test cell,
a step of comparing the expression level of the gene and the expression level of the corresponding gene in a normal tissue or a normal cell, and,
as a result of the comparison, a step of judging as to whether or not the expression level of the gene in the test tissue or the test cell is significantly larger than the expression level of the gene in the normal tissue or the normal cell.


SEQUENCE LISTING

<110>  BANYUPharmaceutical co.,ltd.

<120>  Method of evaluating compound that uses RSK1

<130>  BY0074A

<150>  JP2005-166412
<151>  2005-06-07

<160>  4

<170>  PatentIn version 3.1

<210>  1
<211>  3199
<212>  DNA
<213>  Homo sapiens

<400>  1
gccgaagtgc tagtgccgcg gcggcggcgg cggacggccc agccggagcg cgaggggctc      60

gggggggcgc ggcggttcgg gtcgcagagc cagggacccc aggacccggg aggcggcgca     120

gccggggccg ccggaggagc gcgggtgacc tggcggcggc gagatgccgc tcgcccagct     180

caaggagccc tggccgctca tggagctagt gcctctggac ccggagaatg gacagacctc     240

aggggaagaa gctggacttc agccgtccaa ggatgagggc gtcctcaagg agatctccat     300

cacgcaccac gtcaaggctg gctctgagaa ggctgatcca tcccatttcg agctcctcaa     360

ggttctgggc cagggatcct ttggcaaagt cttcctggtg cggaaagtca cccggcctga     420

cagtgggcac ctgtatgcta tgaaggtgct gaagaaggca acgctgaaag tacgtgaccg     480

cgtccggacc aagatggaga gagacatcct ggctgatgta aatcacccat tcgtggtgaa     540

gctgcactat gccttccaga ccgagggcaa gctctatctc attctggact tcctgcgtgg     600

tggggacctc ttcacccggc tctcaaaaga ggtgatgttc acggaggagg atgtgaagtt     660

ttacctggcc gagctggctc tgggcctgga tcacctgcac agcctgggta tcatttacag     720

agacctcaag cctgagaaca tccttctgga tgaggagggc cacatcaaac tcactgactt     780

tggcctgagc aaagaggcca ttgaccacga gaagaaggcc tattctttct gcgggacagt     840

ggagtacatg gcccctgagg tcgtcaaccg ccagggccac tcccatagtg cggactggtg     900

gtcctatggg gtgttgatgt ttgagatgct gacgggctcc ctgcccttcc aggggaagga     960

ccggaaggag accatgacac tgattctgaa ggcgaagcta ggcatgcccc agtttctgag    1020

cactgaagcc cagagcctct tgcgggccct gttcaagcgg aatcctgcca accggctcgg    1080

ctccggccct gatggggcag aggaaatcaa gcggcatgtc ttctactcca ccattgactg    1140

gaataagcta taccgtcgtg agatcaagcc acccttcaag ccagcagtgg ctcagcctga    1200

tgacaccttc tactttgaca ccgagttcac gtcccgcaca cccaaggatt ccccaggcat    1260

cccccccagc gctggggccc atcagctgtt ccggggcttc agcttcgtgg ccaccggcct    1320

gatggaagac gacggcaagc ctcgtgcccc gcaggcaccc ctgcactcgg tggtacagca    1380

actccatggg aagaacctgg tttttagtga cggctacgtg gtaaaggaga caattggtgt    1440

gggctcctac tctgagtgca agcgctgtgt ccacaaggcc accaacatgg agtatgctgt 1500
caaggtcatt gataagagca agcgggatcc ttcagaagag attgagattc ttctgcggta 1560
tggccagcac cccaacatca tcactctgaa agatgtgtat gatgatggca aacacgtgta 1620
cctggtgaca gagctgatgc ggggtgggga gctgctggac aagatcctgc ggcagaagtt 1680
cttctcagag cgggaggcca gctttgtcct gcacaccatt ggcaaaactg tggagtatct 1740
gcactcacag ggggttgtgc acagggacct gaagcccagc aacatcctgt atgtggacga 1800
gtccgggaat cccgagtgcc tgcgcatctg tgactttggt tttgccaaac agctgcgggc 1860
tgagaatggg ctcctcatga caccttgcta cacagccaac tttgtggcgc ctgaggtgct 1920
gaagcgccag ggctacgatg aaggctgcga catctggagc ctgggcattc tgctgtacac 1980
catgctggca ggatatactc catttgccaa cggtcccagt gacacaccag aggaaatcct 2040
aacccggatc ggcagtggga agtttaccct cagtggggga aattggaaca cagtttcaga 2100
gacagccaag gacctggtgt ccaagatgct acacgtggat ccccaccagc gcctcacagc 2160
taagcaggtt ctgcagcatc catgggtcac ccagaaagac aagcttcccc aaagccagct 2220
gtcccaccag gacctacagc ttgtgaaggg agccatggct gccacgtact ccgcactcaa 2280
cagctccaag cccacccccc agctgaagcc catcgagtca tccatcctgg cccagcggcg 2340
agtgaggaag ttgccatcca ccaccctgtg aggcaccagg gcattcgggc cacagggcgg 2400
tgctagcttg acacagtcag catgcttccc agagggagca ggccggaacc acagggccag 2460
agggagctgg aacccgaggg gccggggaag ctgccagccc agaacacccc taatgagggt 2520
gtgagaagtg ccttctcctt ccccaggatg gactcttctc ggctcaggct ctgctggtgg 2580
aaagcgattc actgtataaa ctttttttta tgaaaaaaat ggcatcaacc accatggatt 2640
tttacaagat ccatttgcct ttctgggagc agaaacagcc attgcggccc caggagggga 2700
actgagtcac gctggggctc tctgagactc tttagagcag ctttgggatc ccaccctggg 2760
gacccccacg attggccacc tgtagccatc tgcacacacc tccgagacag tccagtgtca 2820
cctctctcag agcatctggc tgtttagcag aactcattct atccccaatc agctcctttt 2880
ccgttctgtt ctgctgggag ttctagaacc acttcctgct acaggagggg tctcatgtcc 2940
tgctggcttc cagcttcagg caccagcatc caccttggct ctgccagtgg atcccctgcg 3000
gtcaggctgg gcagccccag agagaggatg tggaaagcac tttttggctg acttcatctg 3060
gggttggcaa caggacagag ttcacaggag gccagtgggc gggccatgag ggacagggtc 3120
ttttttcatt tcttcctcag ctggttactc agggttcatc tgtccatggc ctttctaata 3180
aactgttgag ttgaagcac 3199

<210> 2
<211> 21
<212> RNA
<213> synthetic oligonucleotide

<400> 2
cugucaaggu cauugauaatt 21

<210>  3
<211>  21
<212>  RNA
<213>  synthetic oligonucleotide

<400>  3
ccaaggaccu gguguccaatt                                                    21


<210>  4
<211>  21
<212>  RNA
<213>  synthetic oligonucleotide

<400>  4
cagugacaca ccagaggaatt